# EUROPEAN PATENT SPECIFICATION

(11) **EP 0 974 326 B1**
(45) Date of publication and mention of the grant of the patent: **16.02.2005**
(21) Application number: 98113867.0
(22) Date of filing: 24.07.1998
(51) Int. Cl.: A61F 13/58

(54) **Diaper Closure system**
Windelverschlusssystem
Système de fermeture de couche

(43) Date of publication of application: 26.01.2000
(73) Proprietor: MINNESOTA MINING AND MANUFACTURING COMPANY, St. Paul, Minnesota 55133-3427 (US)
(72) Inventor: Loescher, Claus, 41468 Neuss (DE); Wilhelm, Stefan c/oMinnesota Mining&ManufacturinCo, St. Paul Minnesota 55 144-1000 (US)
(74) Representative: Wilhelm, Stefan

(56) References cited:
- EP-A- 0 321 232
- WO-A-97/28774
- US-A- 5 605 729

## Description

### Field of the invention

The present invention relates to a mechanical closure system for an absorbent article and, particularly, for a disposable diaper. The closure system comprises a mechanical closure tape tab laminate or a precursor of such tape tab laminate and a female fastening element whereby the mechanical closure tab laminate comprises a male mechanical fastening element and the female fastening element, which is attachable to the waistband portion of the diaper, is engagable with the male fastening element. The closure system
- allows to fasten the disposable article onto the body of a person during use (closure feature), whereby the tab laminate is designed
- to allow for securing of the soiled diaper after use for disposal (disposability feature), and
- not to pop open when folded onto the inside surface of the diaper prior to use (anti-flagging feature).

The present invention also relates to an absorbent article comprising said closure system.

### Background of the invention

Closure systems for absorbent articles having some or all of the above features, are described in the art.

EP 0,324,578 discloses a closure system for a disposable garment such as a diaper comprising a loop fastener means in the waistband portion of the garment comprising a multiplicity of loops and a pair of elongate polymeric tab assemblies which are attached to the opposite end of the garment. The loop fastener means includes a backing material and has a plurality of through stitches formed with polymeric strands by a stitch-knitting machine. The tab assemblies comprise a hook fastening element which is engagable with the loop fastener means and thus allows to secure the garment to the wearer's body during use. Each tab assembly further comprises a one-sided pressure-sensitive adhesive tape tab which is attached at one of its ends to the tab assembly. The pressure-sensitive adhesive layer of such tape tab which faces inwardly is releasably adhered to the tab assembly. When the garment is soiled and rolled up for disposal the tape tab can be folded back thereby exposing the pressure-sensitive adhesive layer which can be adhered to the outside surface of the diaper. The closure system of EP 0,324,578 thus provides a mechanical closure feature and a disposability feature but no anti-flagging feature.

EP 0,321,232 discloses a disposable absorbent article comprising a closure system having tape tabs which are attachable to a landing member in order to secure the diaper to an individual. The tape tabs have on their so-called connective portion a mechanical fastening element such as a hook fastening element and an exposed adhesive fastening surface. The landing member comprises a loop fastening element complementary with the hook fastening element on the tape tab. During use the absorbent article is drawn up between the wearer's legs and the tape tabs are attached to the landing member thereby forming a combination mechanical and adhesive closure mechanism. The exposed adhesive fastening surface on the tape tab secures its connective portion to the inside surface of the disposable absorbent article and prevents the tape tab from popping open when handling semi-finished absorbent articles during the process of manufacturing or when storing, packaging or transporting the finished absorbent article. The exposed adhesive fastening surface thus provides for an anti-flagging feature and can furthermore adhere to the outside surface of the diaper when it is rolled up for disposal (disposability feature). The exposed adhesive fastening surface of the tape tab of EP 0,321,232 has on the other hand the disadvantage that it can become contaminated with fiber elements upon adhesion to the loop fastening element on the landing member. Such contamination reduces the tackiness of the exposed adhesive fastening element and adversely affects the disposability feature. The loop material used in EP 0,321,232 may be manufactured from a wide range of materials to provide fiber elements such as, for example, nylon, polyester or propylene or a combination thereof. A specific material mentioned is Scotchmate® nylon woven loop # SJ 3401 available from Minnesota Mining and Manufacturing (3M) Company, Saint Paul, U.S.A.. Closure systems comprising a tape tab laminate having a hook fastening element and an exposed adhesive fastening surface, and a female fastening element being engagable with said hook fastening element, are also disclosed in the European patent application EP 98 104 590.9 filed by the present Applicant on March 13, 1998, or in EP 0,780,109.

It was therefore an object of the present invention to provide a closure system for disposable absorbent articles which allows for secure fastening of the diaper to the wearer's body and which provides both a disposability and an anti-flagging feature without having the shortcomings of the closure systems disclosed in the state of the art. Other objects of the present invention are evident from the following description of the invention.

### Brief description of the invention

The present invention relates to a closure system comprising a mechanical closure tape tab laminate 10 or a precursor of such tape tab laminate, and a female fastening element 100 or a precursor of such female fastening material, for an absorbent article, particularly for a disposable diaper 1, the mechanical closure tape tab laminate 10 comprising a fastening tab 11 having a backing 13 bearing an adhesive layer 12, the mechanical closure tape tab laminate 10 being attachable to the outside surface 3 of a first end region 7 of the diaper 1 or between the outside surface 3 and the inside surface 2 of the edge portion 6 of said first end region 7 of the diaper 1 through its proximal end 17 which is adjacent to its free end 18, the free end 18 of the mechanical closure tape tab laminate 10 further comprising a male mechanical fastening element 15, the mechanical closure tape tab laminate 10 having at least one exposed adhesive fastening surface 19 on the free end 18 of the fastening tape 11, the female fastening element 100 comprising a backing 101 bearing on a first major surface a fibrous web structure 102 having a release-treated exposed surface 103, the fibrous web structure 102 being capable of engaging the male fastening element 15 of the mechanical closure tape tab laminate 10, the female fastening element being attachable to the outside surface 3 of a second end region 8 of the diaper 1 opposite to said first end region 7 through a second major surface of the backing 101 to form a landing zone for the male fastening element 15 of the mechanical closure tape tab laminate 10, the release-treatment of the exposed surface 103 of the fibrous web structure 102 being selected so that the 90° peel adhesion of the exposed adhesive fastening surface 19 from the exposed surface 103 of the female fastening element 100 as measured according to the modified FINAT test method no. 2 described in the test section, is less than 1.2 N/25 mm.

The present invention also refers to a closure system comprising a mechanical closure tape tab laminate 10 or a precursor of such tape tab laminate, and a female fastening element 100 or a precursor of such female fastening material, for an absorbent article, particularly for a disposable diaper 1, the mechanical closure tape tab laminate 10 comprising a fastening tab 11 having a backing 13 bearing an adhesive layer 12, the mechanical closure tape tab laminate 10 being attachable to the outside surface 3 of a first end region 7 of the diaper 1 or between the outside surface 3 and the inside surface 2 of the edge portion 6 of said first end region 7 of the diaper I through its proximal end 17 which is adjacent to its free end 18, the free end 18 of the mechanical closure tape tab laminate 10 further comprising a male mechanical fastening element 15, the mechanical closure tape tab laminate 10 further comprising a secondary tape tab 30 having a backing 31 bearing an adhesive layer 32, the secondary tape tab 30 being attached to the free end 18 of the mechanical closure tape tab laminate 10 by means of the adhesive layer 32, by means of the adhesive layer 12 and/or by means of one or more tertiary tape tabs 40 having a backing 41 and an adhesive layer 42, the secondary tape tab 30 being attachable to the inside surface 2 of the diaper 1 by means of the adhesive layer 32 and/or by means of one or more tertiary tape tabs 40, the mechanical closure tape tab laminate 10 having at least one exposed adhesive fastening surface 19 on the free end 18 of the fastening tape 11, on the secondary tape tab 30 and/or on one or more of the tertiary tape tabs 40, the female fastening element 100 comprising a backing 101 bearing on a first major surface a fibrous web structure 102 having a release-treated exposed surface 103, the fibrous web structure 102 being capable of engaging the male fastening element 15 of the mechanical closure tape tab laminate 10, the female fastening element being attachable to the outside surface 3 of a second end region 8 of the diaper I opposite to said first end region 7 through a second major surface of the backing 101 to form a landing zone for the male fastening element 15 of the mechanical closure tape tab laminate 10, the release-treatment of the exposed surface 103 of the fibrous web structure 102 being selected so that the 90° peel adhesion of the exposed adhesive fastening surface 19 from the exposed surface 103 of the female fastening element 100 as measured according to the modified FINAT test method no. 2 described in the test section, is less than 1.2 N/25 mm.

The present invention also refers to a disposable absorbent article comprising any of such closure systems.

The present invention furthermore refers to a method of decreasing in a closure system comprising a mechanical closure tape tab laminate 10 or a precursor of such tape tab laminate, said tape tab laminate 10 having at least one exposed adhesive fastening surface 19, and a female fastening element 100 or a precursor of such female fastening material, said female fastening element 100 comprising a fibrous web structure 102 having an exposed surface 103, the contamination of said exposed adhesive fastening surface 19 with fibers upon adhering the mechanical closure tape tab laminate 10 to the female fastening element 100, the mechanical closure tape tab laminate 10 comprising a fastening tab 11 having a backing 13 bearing an adhesive layer 12, the mechanical closure tape tab laminate 10 being attachable to the outside surface 3 of a first end region 7 of the diaper 1 or between the outside surface 3 and the inside surface 2 of the edge portion 6 of said first end region 7 of the diaper 1 through its proximal end 17 which is adjacent to its free end 18, the free end 18 of the mechanical closure tape tab laminate 10 further comprising a male mechanical fastening element 15, the mechanical closure tape tab laminate 10 having said at least one exposed adhesive fastening surface 19 on the free end 18 of the fastening tape 11, the female fastening element 100 comprising a backing 101 bearing on a first major surface said fibrous web structure 102 having said exposed surface 103, the fibrous web structure 102 being capable of engaging the male fastening element 15 of the mechanical closure tape tab laminate 10, the female fastening element 100 being attachable to the outside surface 3 of a second end region 8 of the diaper 1 opposite to said first end region 7 through a second major surface of the backing 101 to form a landing zone for the male fastening element 15 of the mechanical closure tape tab laminate 10, said method for decreasing of the contamination of the exposed adhesive fastening surface 19 with fibers upon adhering the mechanical closure tape tab laminate 10 to the female fastening element 100 comprising release-treating of the exposed surface 103 of the fibrous web structure 102, a release control component being applied to the fibrous web structure 102 during such release-treatment being selected so that the 90° peel adhesion between the exposed adhesive fastening surface 19 and the exposed release-treated surface 103 of the female fastening element 100 as measured according to the modified FINAT test method no. 2 described in the test section, is less than 1.2 N/25 mm.

The present invention furthermore refers to a method of decreasing in a closure system comprising a mechanical closure tape tab laminate 10 or a precursor of such tape tab laminate, said tape tab laminate 10 having at least one exposed adhesive fastening surface 19, and a female fastening element 100 or a precursor of such female fastening material, said female fastening element 100 comprising a fibrous web structure 102 having an exposed surface 103, the contamination of said exposed adhesive fastening surface 19 with fibers upon adhering the mechanical closure tape tab laminate 10 to the female fastening element 100, the mechanical closure tape tab laminate 10 comprising a fastening tab 11 having a backing 13 bearing an adhesive layer 12, the mechanical closure tape tab laminate 10 being attachable to the outside surface 3 of a first end region 7 of the diaper 1 or between the outside surface 3 and the inside surface 2 of the edge portion 6 of said first end region 7 of the diaper 1 through its proximal end 17 which is adjacent to its free end 18, the free end 18 of the mechanical dosure tape tab laminate 10 further comprising a male mechanical fastening element 15, the mechanical closure tape tab laminate 10 further comprising a secondary tape tab 30 having a backing 31 bearing an adhesive layer 32, the secondary tape tab 30 being attached to the free end 18 of the mechanical closure tape tab laminate 10 by means of the adhesive layer 32, by means of the adhesive layer 12 and/or by means of one or more tertiary tape tabs 40 having a backing 41 and an adhesive layer 42, the secondary tape tab 30 being attachable to the inside surface 2 of the diaper 1 by means of the adhesive layer 32 and/or by means of one or more tertiary tape tabs 40, the mechanical closure tape tab laminate 10 having said at least one exposed adhesive fastening surface 19 on the free end 18 of the fastening tape 11, on the secondary tape tab 30 and/or on one or more of the tertiary tape tabs 40, the female fastening element 100 comprising a backing 101 bearing on a first major surface said fibrous web structure 102 having said exposed surface 103, the fibrous web structure 102 being capable of engaging the male fastening element 15 of the mechanical closure tape tab laminate 10, the female fastening element 100 being attachable to the outside surface 3 of a second end region 8 of the diaper 1 opposite to said first end region 7 through a second major surface of the backing 101 to form a landing zone for the male fastening element 15 of the mechanical closure tape tab laminate 10, said method for decreasing of the contamination of the exposed adhesive fastening surface 19 with fibers upon adhering the mechanical closure tape tab laminate 10 to the female fastening element 100 comprising release-treating of the exposed surface 103 of the fibrous web structure 102, a release control component being applied to the fibrous web structure 102 during such release-treatment being selected so that the 90° peel adhesion between the exposed adhesive fastening surface 19 and the exposed release-treated surface 103 of the female fastening element 100 as measured according to the modified FINAT test method no. 2 described in the test section, is less than 1.2 N/25 mm.

### Brief description of the figures

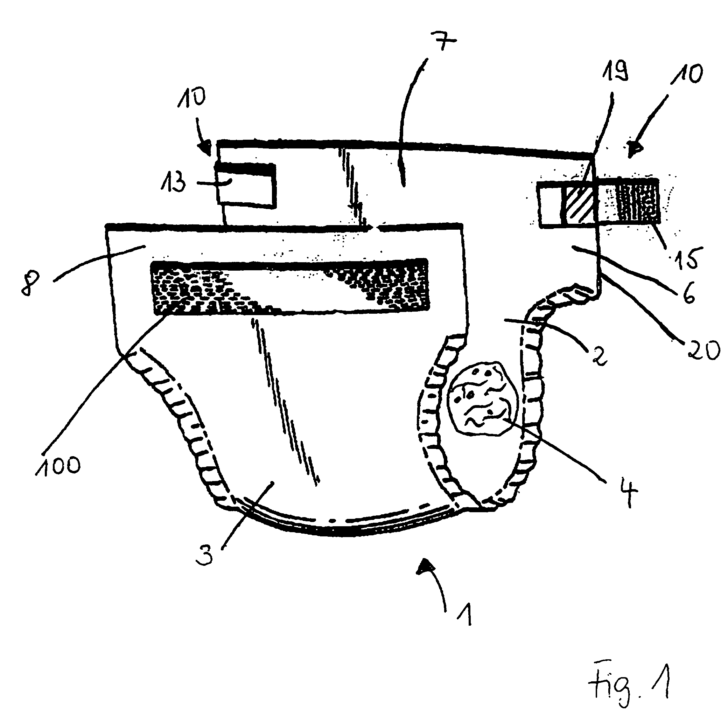

Fig. 1 is a perspective representation of a diaper 1 having an absorbent core 4 between an inside surface 2 and an outside surface 3, mechanical closure tape tab laminates 10 anchored to the edge portions 6 of the first end region 7 of the diaper 1 and a female fastening element 100 secured to the outside surface 3 of the second end region 8 of the diaper to form a landing zone for the male fastening element 15 of the mechanical closure tape tab laminates.

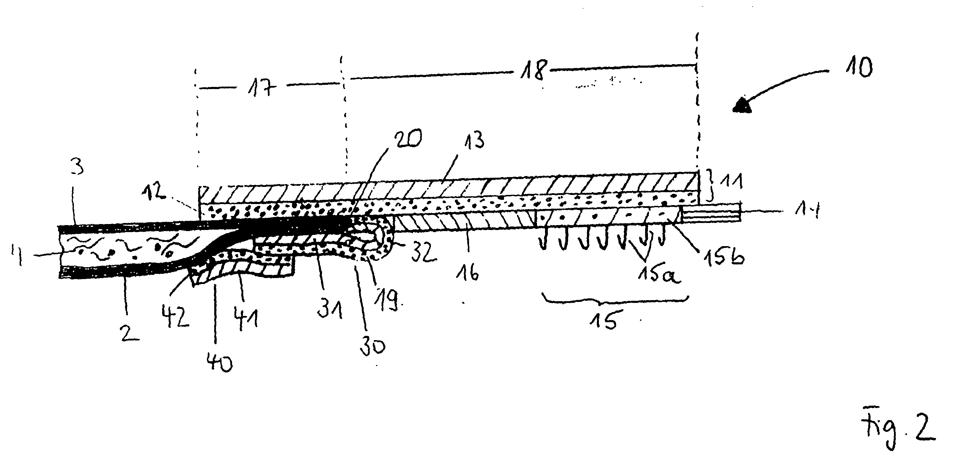

Fig. 2 is a cross-sectional view of a mechanical closure tape tab laminate 10 useful in the present invention being attached to the edge portion 6 of the first end region 7 of the diaper 1, said mechanical closure tape tab laminate 10 composing a backing 13 bearing a continuous adhesive layer 12, a cover film 16, a hook fastening element 15, a fingerlift 14, a secondary tape tab 30 which is distally adhered with its adhesive layer 32 to the adhesive layer 12 and which is proximally adhered to the inside surface 2 of the diaper 1 through a tertiary tape tab 40, the secondary tape tab 30 providing an exposed adhesive fastening surface 19 above the inside surface 2 of the diaper 1.

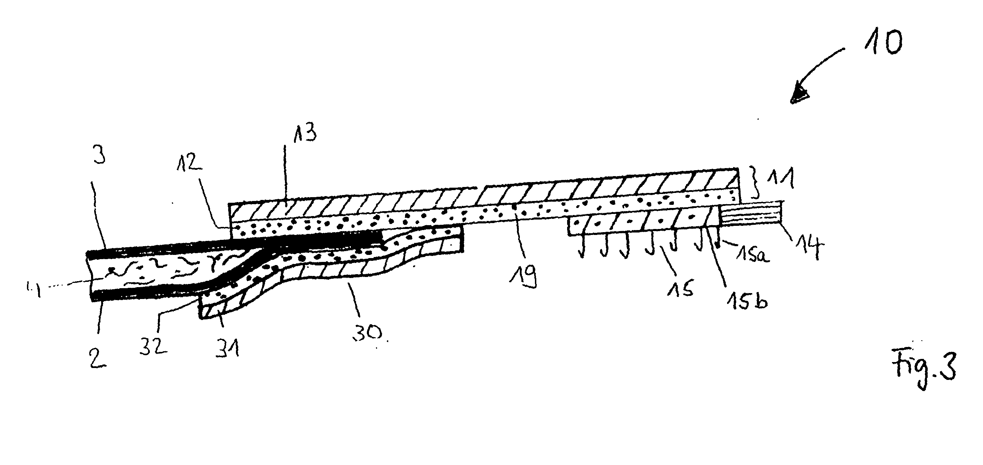

Fig. 3 is a cross-sectional view of a mechanical closure tape tab laminate 10 useful in the present invention being attached to the edge portion 6 of the first end region 7 of the diaper 1, said mechanical closure tape tab laminate 10 comprising a backing 13, bearing a continuous adhesive layer 12, a hook fastening element 15, a fingerlift 14, a secondary tape tab 30 which is distally adhered with its adhesive layer 32 to the adhesive layer 12 and proximally adhered with its adhesive layer 32 to the inside surface 2 of the diaper 1, the adhesive layer 12 providing an exposed adhesive fastening surface 19 between the distal end of the secondary tape tab 30 and the proximal end of the hook fastening element 15.

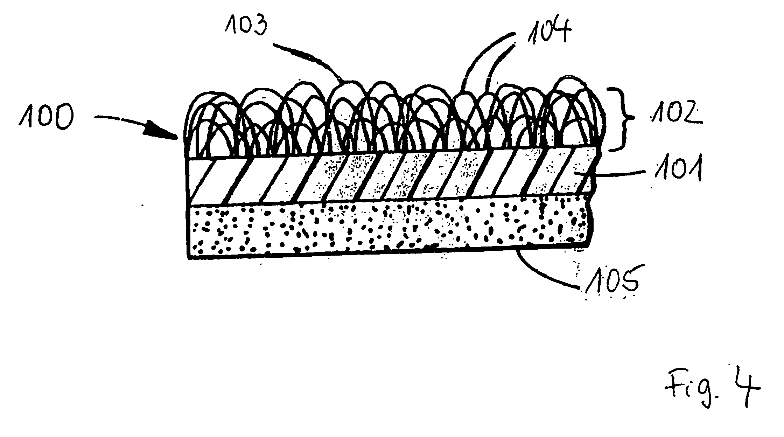

Fig. 4 is a cross sectional view of a female fastening element 100 comprising a fibrous web structure comprising a multitude of upstanding loop elements 14 emanating from and anchored to the first major surface of a backing 101, said backing 101 exhibiting on a second major surface opposite to the first major surface an adhesive layer 105.

### Detailed description of the invention

The mechanical closure systems of the present invention are useful and beneficial when attached to a disposable absorbent article. As used therein, the term "disposable absorbent article" refers to articles which are placed against or in proximity to the body of the wearer to absorb and contain the various exudates discharged from the body and which are intended to be disposed of after single use (i.e., they are not intended to be laundered or otherwise restored or reused).

A preferred embodiment of the disposable absorbent article of the present invention is a diaper 1. As used herein, the term "diaper" refers to a garment generally worn by infants or incontinent persons that is drawn up between the legs and fastened about the waist of the wearer.

Fig. 1 is a partially cut-away perspective representation of a disposable diaper 1. The diaper comprises an absorbent core 4 between an inside surface 2 (commonly also referred to as nonwoven topsheet) and an outside surface 3 (commonly also referred to as nonwoven backsheet). The absorbent core 4 may be any means which is generally compressible, conformable, non-irritating to the wearer's skin, and capable of absorbing and retaining liquids and certain body exudates.

The outside surface 3 of the diaper is impervious to liquids and is preferably manufactured from a thin plastic film or film laminate, although other flexible, liquid impervious materials may also be used. The outside surface 3 prevents the exudates absorbed and contained in the absorbent core 4 from soiling articles which contact the diaper 1 such as bedsheets and undergarments.

The inside surface 2 of the diaper is compliant, soft-feeling, and not-irritating to the wearer's skin. Further, the inside surface 2 is liquid pervious permitting liquids to readily penetrate through its thickness. A suitable inside surface 2 may be manufactured from a wide range of materials such as porous foams, reticulated foams, apertured films, natural fibers (e.g., wood or cotton fibers), synthetic fibers (e.g., polyester or polypropylene fibers) or from a combination of natural and synthetic fibers. Preferably, it is made of a hydrophobic material to isolate the wearer's skin from liquids retained in the absorbent core 4. A suitable inside surface 2 may be, for example, a spunbond or carded polypropylene nonwoven having a basic weight of approximately 15-25 g/m².

The absorbent core 4 may be secured to the outside surface 3 and/or the inside surface 2 by means of, for example, pressure-sensitive adhesives, hot melt adhesives or other adhesives, ultrasonic bonding or heat/pressure sealing The outside surface 3 and the inside surface 2 may be joined to each other directly or indirectly by using an intermediate fixing member to which the outside surface 3 and the inside surface 2 are affixed. The inside surface 2 and the outside surface 3 may be associated together by various means comprising, for example, pressure-sensitive adhesives, hot melt adhesives or other adhesives, ultrasonic bonding and/or heat and/or pressure.

The above description of the diaper 1 is meant to be explanatory only and not limiting. Further details on diapers and their construction are described in the literature and may be taken, for example, from EP 0,529,681, US 4,036,233, EP 0,487,758, WO 96/10,382, US 3,800,796, EP 0,247,855 or US 4,857,067.

The diaper has a closure system comprising
- a pair of a mechanical closure tape tab laminate 10 comprising a male fastening element 15, said tape tab laminates 10 being attached to the edge of the portions 6 of a first end region 7 of the diaper, and
- a female fastening material 100 attached to outside surface 3 of the second end region 8 of the diaper 1 opposite to said first end region 7, to provide a landing zone for the male fastening element 15 of the mechanical closure tape tab laminate 10.

Mechanical closure tape tab laminates 10 which are useful in the present invention comprise a fastening tape tab 11 having a backing 13 bearing an adhesive layer 12.

The backing 13 is preferably selected from a group of materials which is essentially non-elastic and imparts a desirable stiffness to the tape tab laminate 10.

The backing 13 is preferably selected from a group of materials comprising polypropylene, polyvinylchloride, polyethylene terephtalate, polyethylene, polyolefin copolymers or blends of polyolefins such as, for example, a blend of polypropylene and LPDE (low density polyethylene), non-woven, foamed materials and laminates thereof. Laminates comprising elastic materials may also be used. The thickness of the backing 13 preferably is between 50 µm and 500 µm and more preferably between 80 µm and 350 µm.

The exposed surface of the backing 13 preferably has release properties and/or is treated with a release coating so that the backing 13 bearing the adhesive layer 12 can be easily wound up in a roll without requiring a release liner. The release coating may be any of the known materials used for their release properties for adhesives. Preferred types are silicones and modified silicones, the modification including both copolymerization of silicones with other non-release chemical agents or by adding non-silicone materials to the silicone coating solution prior to application to the release base paper. Other release agents such as fluorocarbons and polyvinyl octadecyl carbamate may also be used. The choice of release coating is dependent on the tack, adhesion level, and chemical nature of the adhesive layer 12.

The backing 13 bears an adhesive layer 12 which preferably extends over the whole length of the fastening tape tab 11 but may also cover only part of the backing 13. It is, for example, possible that in the embodiment of the mechanical closure tape tab laminate 10 of Fig. 2, the covering film 16 and the adhesive layer 12 in the area of the covering film 16, are omitted. Non-continuous adhesive layers 12 can be obtained, for example, by strip-coating of the adhesive to the backing 13. The adhesive of the adhesive layer 12 is preferably selected to permanently attach the proximal end 17 of the backing 13 to the outside surface 3 of the diaper 1. Likewise, the adhesive of the adhesive layer 12 is preferably selected to permanently adhere, for example, the male mechanical fastening element 15 to the backing. The adhesive useful for adhesive layer 12 comprises pressure-sensitive adhesives including pressure-sensitive hot-melt adhesives and non-pressure-sensitive adhesives.

Suitable pressure-sensitive adhesives include rubber-based adhesives (also called rubber-resin adhesives) which comprise natural or synthetic rubber materials and typically also tackifying resins in order to render the rubber materials tacky. Preferred examples of rubber resin based pressure-sensitive adhesives are the polystyrene polyisoprene block copolymers tackified with synthetic polyterpene resins or hydrocarbon resins. Suitable pressure-sensitive adhesives furthermore include acrylate-based pressure-sensitive adhesives such as, for example, those disclosed in US Re 24,906 or US 4,710,536. The adhesives mentioned above are given only by way of example, and the person skilled in the art can select other adhesives known in the state of the art. The thickness of the adhesive layer 12 preferably is between 10 and 200 µm and more preferably between 20 and 100 µm.

The male fastening element 15 useful in the present invention comprises a multiplicity of flexible hook elements 15a emanating from a backing 15b. The hook elements 15a preferably comprise a stem supported at the backing 15b, and an enlarged hook head positioned at the end of the stem opposite to the backing 15b. The geometrical shape of the hook elements is not particularly limited and comprises the variety of shapes described in the literature.

Mushroom-shaped hook fastening elements are described, for example, in WO 94/23,610, US 5,077,870, and 3,718,725 whereas US 3,594,865, US 4,884,339, US 5,315,740, US 4,794,028 and US 5,620,769 disclose J-shaped hook fastening elements. A wide variety of sizes and shapes of hook elements are described in WO 94/23,610, WO 92/04,839 and U.S. serial no. 08/723,632 filed by the present Applicant. US 4,984,060 describes J- and T-shaped hooks as well as more complicated geometrical forms. The geometry of the hook elements 15 described above is given by way of example only, and other shapes can be used as well.

The size of the hook elements 15a can vary over a broad range. For use in disposable absorbent articles such as diapers, the hook elements 15a preferably are of essentially uniform height, preferably from about 0.10 to 1.3 mm in height, and more preferably from about 0.2 to 0.5 mm in height. The stems of the hook element 15a have a diameter adjacent to the hook head of preferably from 0.07 to 0.7 mm and more preferably from about 0.1 to 0.3 mm. The hook heads preferably project radially past the stems in at least one direction by, on average, about 0.02 to 0.25 mm. The maximum thickness of the hook heads as measured in a direction parallel to the axis of the stems between their outer and inner surfaces, preferably is from about 0.01 to 0.3 mm and more preferably from about 0.02 to 0.1 mm. The hook heads preferably have an average maximum diameter to average maximum thickness ratio preferably from 1.5:1 to 12:1, and more preferably from 2.5:1 to 6:1. The hook density preferably is between 100 and 500 hook elements/cm².

The male fastening element 15 useful in the present invention can be prepared by weaving techniques or extrusion molding with extrusion molding being preferred. Virtually any orientable thermoplastic resin that is suitable for extrusion molding may be used. Preferable thermoplastic resins include, for example, polyesters such as poly(ethylene terephthalate), polyamides such as nylon, poly(styrene-acrylonitrile), poly(acrylonitrile-butadione-styrene), polyolefins such as polypropylene, polyolefin copolymers and plasticized polyvinyl chloride. To have good flexibility and strength, the backing layer 15b of the male fastening element 15 preferably is a film from 20 µm to 500 µm in thickness, and more preferably is from 60 µm to 300 µm thick, especially when the male fastening element 15 is made of polypropylene or a copolymer of propylene and ethylene. For some applications, a stiffer backing can also be used. An example of a male fastening element 15 suitable for use in the present invention, is commercially available from 3M Company, St. Paul, U.S.A. under the trade designation KN-2396.

The mechanical closure tape tab laminates 10 useful in the present invention may optionally comprise a fingerlift 14 which is attached to the distal end portion of the free end 18 of the mechanical closure tape tab laminate 10 to allow for easier handling. The fingerlift 14 may partly extend over the distal end of the backing 13 as is shown, for example, in Fig. 2. In another embodiment, the backing 13 may extend over the distal end of the adhesive layer 12 and, optionally, be folded back onto the adhesive layer to provide for a fingerlift 14. The fingerlift 14, if present, preferably has an extension in cross-direction of typically between 3 and 10 mm, a thickness of between 5 and 200 µm and is preferably prepared from a group of materials comprising polymer films, non-wovens and laminates thereof.

The fastening tape tab 11 of the mechanical closure tape tab laminate 10 is secured to the edge portion 6 of a first end region 7 of the diaper through its proximal end 17 whereby several configurations are possible. In a first anchoring mode which is termed as peel mode type attachment, the fastening tape tab 11 is adhered to the outside surface 3 of the diaper through the proximal end of the adhesive layer 12. Peel mode type anchored tab laminates 10 may loosen when attaching the diaper to the wearer's body and during use so that this type of attachment is usually less preferred.

An additional anchorage of the tab laminate 10 to the diaper 1 is obtained by using a secondary tape tab 30 having a backing 31 bearing an optionally continuous adhesive layer 32, said secondary tape tab 30
- being attached to the free end 18 of the mechanical closure tape tab laminate 10 by means of it's adhesive layer 32, by means of the adhesive layer 12 of the fastening tape tab 11 and/or by means of one or more tertiary tape tabs 40, and
- being attachable to the inside surface 2 of the diaper 1 by means of its adhesive layer 32 and/or by means of one or more tertiary tape tabs, 40

Applying a secondary tape tab 30 results in the so-called Y-bond anchoring mode which is preferred and allow for especially reliably securing of the tape tab laminate 10 to the diaper. Alternatively, the proximal end 17 of the fastening tape tab 11 can be bonded in between the outside surface 3 and the inside surface 2 of the edge portion 6 of the first end region 7 of the diaper.

The secondary tape tab 30 comprises an optionally continuous adhesive layer 32 and a backing 31. The backing 31 may be manufactured from a wide range of materials such as polymer films or paper films. The backing is preferably selected from a group of materials comprising polypropylene, polyvinylchloride, polyethylene terephthalate, polyethylene, polyolefin copolymers or blends of polyolefins such as, for example, a blend of polypropylene and LDPE (low density polyethylene), non-wovens, foamed materials and laminates thereof. The adhesive of adhesive layer 32 can be selected from the adhesives suitable for adhesive layer 12 described above, although the adhesive of adhesive layer 32 preferably is less aggressive than the adhesive of adhesive layer12.

It is essential in the closure system of the present invention that the mechanical closure tape tab laminate 10 comprises an exposed adhesive fastening surface 19. This can be effected in various ways, and some preferred, non-limiting constructions will be described in some detail below.

The mechanical closure tape tab laminate 10 shown in Fig. 2, is described in some detail in European Patent Application EP 98 104 590.9 filed by the present Applicant on March 13, 1998. The mechanical closure tape tab laminate 10 comprises a fastening tape tab 11 comprising a backing 13 bearing an adhesive layer 12. The fastening tape tab 11 is proximally adhered to the outside surface 3 of the edge portion 6 of the first end region 7 of the diaper 1 through adhesive layer 12. The tape tab laminate 10 comprises a secondary tape tab 30 which is positioned on the inside surface 2 of the diaper 1 so that its adhesive layer 32 is exposed. The secondary tape tab 30 is proximally adhered to the inside surface 2 of the diaper 1 by means of a tertiary tape tab 40 comprising a backing 41 and an adhesive layer 42. The distal end portion of the secondary tape tab 30 which distally extends beyond the longitudinal edge 20 of the diaper 1 is folded back upon itself so as to distally adhere the backing 31 of the secondary tape tab 30 through adhesive layer 12 to the fastening tape tab 11. The mechanical closure tape tab laminate 10 further comprises at its distal end a male fastening element 15 and a fingerlift 14 which are laminated to the adhesive layer 12 of the fastening tape tab 11. The portion of the adhesive layer 12 between the distal end of the secondary tape tab 30 and the proximal end of the male fastening element 15 is covered by a cover film 16.

In the tab laminate 10 of Fig. 2 the exposed adhesive fastening surface 19 is provided by the adhesive layer 32 of the secondary adhesive tape tab 30. When folding the free end 18 of the mechanical tab laminate 10 onto the inside surface 2 of the diaper for storage prior to use, the exposed adhesive surface 19 releasably adheres to the cover film 16 thus providing an anti-flagging feature. When rolling up the diaper after use, the exposed adhesive fastening surface 19 can be adhered to the outside surface 3 of the diaper 1 thus securing the diaper 1 for disposal.

The cover film 16 and/or the adhesive of the adhesive layer 32 of the secondary tape tab are selected so that the free end 18 of the tab laminate 10 can be releasably adhered to the exposed adhesive fastening surface 19 to provide for an anti-flagging feature. The covering film 16 is preferably selected from a group of materials comprising polyolefin homopolymers or copolymers such as, for example, homopolymers or copolymers of ethylene and/or propylene, polyesters or blends comprising such polymers. The thickness of the covering film 16 preferably is between 50 and 100 µm and more preferably is between 10 and 40 µm.

The material of the backing 41 and of the adhesive layer 42 of the tertiary tape tab 40 is preferably selected using the guidelines given above for the backing 13 and the adhesive layer 12, respectively.

European Patent Application EP 98 104 590.9 describes a variety of related tape constructions which comprise an exposed adhesive layer 19 above the inside surface of the diaper. The construction shown in Fig. 13 of EP 98 104 590 0 employs, for example, a first and second respectively, tertiary tape tab 40.

Another class of mechanical closure tape tab laminates 10 which is useful in the present invention is disclosed in EP 0,321,232. Fig. 3 of the present specification shows the construction of an example of such mechanical closure tape tab laminate which comprise a fastening tape tab 11 having a backing 13 bearing an adhesive layer 12, and a secondary tape tab 30 having an adhesive layer 32 and a backing 31. The fastening tape tab 11 is adhered through the adhesive layer 12 of its proximal end 17 to the outside surface 3 of the diaper 1. The secondary tape tab 30 is adhered through the proximal end portion of its adhesive layer 32 to the inside surface 2 of the diaper and through the distal end portion of its adhesive layer 32 to the adhesive layer 12 of the fastening tape thus providing a Y-bond anchoring mode. A male fastening element 15 and a fingerlift 14 are laminated to the distal end portion of the fastening tape 11. The adhesive layer 12 between the distal end of the secondary tape tab 30 and the proximal end of the male fastening element 15 is exposed and forms the exposed adhesive fastening surface 19.

The backing 31 of the secondary tape tab 30 is selected so that the exposed adhesive fastening surface 19 when folding the free end 18 of the mechanical closure tape tab laminate 10 of Fig. 3 onto the inside surface 2 of the diaper 1, releasably adheres to the backing 31 of the secondary tape tab 30 and prevents the free end 18 of the tab laminate 10 from popping open (anti-flagging feature). The backing 31 may optionally be release-treated. The exposed adhesive fastening surface 19 can also be used to secure the soiled diaper when it is rolled up for disposal (disposability feature).

EP 0,321,232 also describes some modified tab laminate constructions and suggests, for example, that the exposed adhesive layer 19 be split into two portions being arranged proximally and distally, respectively, from the male fastening element 15.

The embodiments of the tape tab laminate 10 described above are given by way of example only and other constructions can be used as well. Tape tab laminates 10 which are useful in the present invention are also described, for example, in EP 0,780,109.

The closure system of the present invention comprises as a second part a female fastening material 100 comprising a sheet-like fibrous web structure 102 capable of engaging the male fastening element 15 of the mechanical closure tape tab laminate 10, said web structure 102 being anchored to the first major surface of a backing 101. A second major surface of the backing 101 optionally bears an adhesive layer 105 for attaching the female fastening element to the second end region 8 of the diaper 1 to form a landing zone for the male fastening element 15.

If can be seen from Fig. 1 that the female fastening element 100 is usually applied to the second end region 8 on the outside surface 3 of the diaper 1. The female fastening element 100 thereby preferably extends over a major portion of the second end region 8 so that the mechanical closure tape tab laminate 10 is attached through its male fastening element 15 to the female fastening element 100 during normal use in a way that the exposed adhesive fastening surface 19 of the tape tab laminate 10 contacts the female fastening element 100. The term "during normal use" means that the size of the diaper 1 is adjusted to the size of the wearer and that the diaper is properly drawn up between the legs of the wearer. With the exposed adhesive surface 19 of the tab laminate 10 being attached to the female fastening element 100, formation of an adhesive closure mechanism between the outside surface 3 of the diaper 1 and the exposed adhesive fastening surface 19 of the tape tab laminate 10 is avoided. This is desirable because upon releasing the male fastening element 15 of the tape tab laminate 10 from the female fastening element 100, such adhesive closure mechanism would frequently result in deforming and/or tearing of the material forming the outside surface 3 of the diaper 1 such as, for example, a polyolefine film.

The sheet-like fibrous web structure 102 shown in the cross-sectional view of Fig.4 can comprises a multitude of upstanding loops 104 anchored to and emanating from a first major surface of a fibrous base layer. This structure may be prepared, for example, by weaving a base fabric containing supplementary threads to form the loops or by knitting loops into a fabric. Other methods of preparing sheet-like fibrous web structures 102 comprising a multitude of upstanding loops, include warp knitting weft insertion knitting, circular knitting, stitching or methods for making non-woven structures such as, for example, pleating, corrugating or embossing.

Illustrative examples of sheet-like fibrous web structures 102 comprising a multitude of loop elements 104, useful in the present invention include knits such as, for example, tricot knits, warp knits, weft inserted knits or circular knits, stitched materials or non-woven materials. The fibrous web structure 102 typically has a basis weight of about 20 to 80 g/m² and is preferably made of yarns or fibers having a denier per filament of between about 2 and 20. Suitable materials for preparing sheet-like fibrous web structures 102 include, for example, polyolefin, polyester or nylon.

The size of the loop elements 104 can vary over a wide range and is preferably selected to provide for a certain male fastening element 15 with a given geometry a secure mechanical closure mechanism. The hook elements 104 of the fibrous web structure 102 may vary considerably in height but preferably are of essentially uniform height.

The fibrous base layer of the fibrous web structure 102 may form the backing 101 but it is preferred that the backing 101 comprises one or more additional layers attached to a second major surface of the fibrous base layer opposite to a first major, outwardly facing exposed surface 103 (i.e. attached to the backside of the loop structure) in order to impart desirable sheet like properties to the female fastening element 100 and to increase, for example, its tear strength, tensile strength and/or flexibility. One or more additional backing layers 101 are also desirable in order to provide a barrier between the adhesive layer 105 and the loop elements 104 to prevent the adhesive from blocking the loop. Suitable additional backing layers 101 which may be attached to the fibrous base layer include, for example, polymeric films such as polyolefin films or polyester films, paper sheets or non-woven webs. It is disclosed in US 4,761,318 to apply a softenable thermoplastic resin layer to the fibrous base layer to bond together and secure the filaments of the fibrous web structure in order to anchor the loop elements 104, and to add sufficient internal strength and integrity to the female fastening element 100 so that even very porous nonwoven structures can be used. If desired, multilayer backing layers or foamed backing layers may be used. Such additional backing layer or layers may, for example, be laminated or extrusion-or roll-coated to the fibrous base layer.

Sheet-like fibrous web-structures 102 comprising a multitude of loops elements 104 are disclosed, for example, in US 4,624,116, US 4,761,318, US 4,931,343, US 4,973,326, US 5,032,122, WO 92/01,401, US 5,505,729 and US 5,616,394.

Another preferred class of female fastening elements which is useful in the present invention includes essentially loop-less sheet-like fibrous web-structures 102 such as, for example, those disclosed in US 5,326,612. This material comprises a multitude of fibers adhered by interfiber bonds and attached to a backing 101 by individual bonding sites. The fibrous web structure exhibits an essentially planar outwardly facing surface and is sufficiently porous as to allow the complimentary hook fastening element to penetrate said web.

The female fastening element preferably comprises an adhesive layer 105 in order to allow for adhering the female fastening element 100 to the second end region 8 of the diaper 1. Suitable adhesives comprise pressure-sensitive adhesives including pressure-sensitive hot-melt adhesives and non-pressure sensitive adhesives. Pressure-sensitive adhesives which are useful in the present invention can be selected, for example, from the group of adhesives disclosed above for adhesive layer 12 of the tape tab laminate 10. Suitable non-pressure-sensitive adhesives include, for example, softenable thermoplastic resins such as, for example, polyethylene, polypropylene, blends and copolymers thereof, ethylene acrylic acid copolymer, nylon copolymers or ethylene vinyl acetate copolymers.

The adhesive layer 105 of the female fastening element 100 is optional. It is, for example, disclosed in US 4,761,318 to apply a softenable thermoplastic resin as a backing layer 101 to the fibrous web structure 102 and to use this backing 101 for adhering the female fastening element to the outside surface 3 of a diaper 1 by softening the surface of the thermoplastic resin layer opposite to the loop side by exposure to heat and contacting the softened surface with the diaper. It is also possible to apply an adhesive layer 105 to the outside surface of the diaper and to apply the female fastening element 100 to such adhesive layer. The female fastening element 100 may also be directly incorporated into a nonwoven outside surface 3 of the diaper.

It is essential in the present invention that the outwardly facing exposed surface 103 of the fibrous web structure 102 is release-treated so that the 90° peel adhesion of the exposed adhesive fastening surface 19 of the tape tab laminate 10 from the exposed surface 103 when measured according to the modified FINAT test method no. 2 described in the test section below, is less than 1.2 N/25 mm, more preferably not more than 1 N/25mm and especially not more than 0.8 N/25 mm.

It was found by the present inventors that when using a fibrous web structure 102 having a 90° peel adhesion from the exposed adhesive fastening surface 19 of more than 1.2 N /25 mm, the exposed adhesive fastening surface 19 of the tape tab laminate 10 upon contact with the fibrous web structure 102 tends to become contaminated with fibers to an unacceptably high degree. Such contamination reduces the adhesive force of the exposed adhesive fastening surface 19 and may adversely effect the disposability feature, i.e. the adhesive disposal mechanism between the exposed adhesive fastening surface 19 and the outside surface 3 of the diaper 1 formed after the diaper 1 has been rolled up for disposal.

It was also observed that when using a fibrous web structure 102 having a 90° peel adhesion from the exposed adhesion fastening surface 19 of more than 1.2 N/25mm, repeated application of the tape tab laminate 10 to the female fastening element 100 results in a "fuzzying" or "stringing" of the fibrous web structure 102 which adversely effects the mechanical closing mechanism between the male fastening element 15 of the tape tab laminate 10 and the female fastening element 100 and which is also not acceptable from an aesthetic point of view. Opening and re-application of the tape tab laminate 10 to the female fastening element 100 is frequently necessary when securing the diaper to the wearer's body in order to obtain a proper fit and when checking the diaper for exudates.

When using a female fastening element 100 having a release treated outwardly facing surface 103 to provide a 90° peel adhesion measured according to the modified FINAT test method no. 2 described in the test section below, between the exposed adhesive fastening surface 19 and the outwardly facing surface 103 of the female fastening element 100 of less than 1.2 N/25 mm, the contamination of the exposed adhesive fastening surface 19 with fibers is effectively suppressed even on repeated opening and re-application of the mechanical closing mechanism between the hook fastening element 15 and the female fastening element 100. This allows to reduce the aggressiveness of the of the adhesive used to provide the exposed adhesive fastening surface 19 of the tape tab laminate 10 of the present invention as compared to the aggressiveness of the adhesive used in the exposed adhesive fastening surface of a tape tab laminate of a closure system of the prior art. The adhesive of the exposed adhesive fastening surface 19 is preferably selected so that the 90° peel adhesion between such adhesive fastening surface 19 and the exposed surface 103 of the fibrous loop structure 102 when measured according to the modified FINAT test method no 2 described in the test section below, is less than 0.75 N/25 mm, more preferably not more than 0.5 N/25 mm and especially preferably not more than 0.25 N/25 mm.

Reducing of the aggressiveness of the adhesive providing the exposed adhesive fastening surface 19 is desirable. In case of an inadvertent misalignment of the diaper 1 when securing the diaper 1 to the wearer's body, such misalignment resulting in an adhesive bonding mechanism between the exposed adhesive fastening surface 19 and the outside surface 3 of the diaper 1, is less frequently observed to cause tearing of the material forming the outside surface 3 of the diaper when using an adhesive with reduced aggressiveness.

The mechanical closure tape tab laminate 10 used in the closure system of the present invention is preferably selected so that upon application of such tape tab laminate 10 to the diaper 1, the exposed adhesive fastening surface 19 is essentially above the inside surface 2 of the diaper while the mechanical fastening element 15 is essentially positioned at the distal end of the tape tab laminate 10 so that the exposed adhesive fastening surface 19 and the male fastening element 15 are not directly adjacent to each other. An example of a tape tab laminate 10 especially suitable for use in the closure system of the present invention, is shown in Fig. 2 where the distal end of the exposed adhesive fastening surface 19 and the proximal end of the male fastening element 15 are separated by the cover film 16. When handling the diaper 1 and attaching the tape tab laminate 10 to the female fastening element 100 to secure the diaper 1 to the wearer's body, a contamination of the exposed adhesive fastening surface 19 by oily or powdery fingers of the person applying the diaper 1, is typically avoided when using a preferred tape tab laminate 10 comprising a distance between the distal end of the exposed adhesive fastening surface 19 and the proximal end of the male fastening element 15. Contrary to this, in tape tab laminate 10 constructions like those shown in Fig.3 where the distal end of the exposed adhesive fastening surface 19 and the proximal end of the male fastening element 15 are directly adjacent to each other a contamination of the exposed adhesive fastening surface 19 with oil and/or powder during handling of the diaper is sometimes observed. Using tape tab laminates having a distance between the exposed adhesive fastening surface 19 and the male fastening element 15, is especially preferred in tape tab laminates 10 having a reduced tackiness of the adhesive of the exposed adhesive fastening surface 19 so that the 90° peel adhesion between such exposed adhesive fastening surface 19 and the exposed release-treated surface 103 of the fibrous loop structure 102 when measured according to the modified FINAT test method no. 2 described in the test section below, is less than 0.75 N/25 mm, more preferably not more than 0.5 N/25 mm and especially preferably not more than 0.25 N/25 mm.

Female fastening elements 100 having a release treated exposed surface 103 are known. It is disclosed, for example, in US 5,605,729 that a release control component may be incorporated as an additive into the material from which the fibrous web structure 102 is made, or be attached to such a fibrous web structure via graft polymerization. Alternatively, the release control component may be applied as an optionally solvent borne coating to the exposed surface 103 of the female fastening element 100.

The release control component may comprise one or more release control agents. Illustrative examples of incorporation-type release control agents include melt additives such as the fluorochemical graft polymer disclosed in WO 92 15,626. Illustrative examples of surface-treatment type (i e., topical) release agents include urethanes such as disclosed in U.S. 2,532,011, reactive silicones, fluorochemical polymers, epoxysilicones such as are disclosed in U.S. 4,313,988 and U.S. 4,482,687 or radiation curable polyorganosiloxane polyurethane block copolymers such as are disclosed in EP 0,250,248. The materials listed here are intended to be explanatory and not limiting.

Coating of a release control component is preferred. The release control component preferably is coated as a solution comprising between 1-25 wt %, more preferably between 2-15 wt % of one or more release agents; solvents suitable in the present invention comprise, for example, methyl ethyl ketone or heptane. The release coating on the surface 103 of the fibrous loop structure 102 preferably exhibits a dried release coating weight of between 0.05-5 g/m² and more preferably of between 0.2-2 g/m².

Female fastening elements 100 having a release treated exposed surface 103 which are suited in the present invention, are described in some detail in US 5,605,729. A preferred release-treated female fastening element 100 is commercially available as Knitted Loop Tape from Minnesota Mining and Manufacturing Company. St. Paul. USA.

The mechanical closure tape tab laminates 10 described in EP 0,321,232 and EP 0,780,109 have been designed so that a combination of a mechanical and adhesive closure mechanism results upon application of said tape tab laminate 10 to the female fastening element 100. Such combination of a mechanical and adhesive closure mechanism is sometimes also referred to as two-point closure mechanism. It was recognized, however, by the present inventors that closure systems providing a two-point closure mechanism, are typically also characterized by a contamination of the exposed adhesive fastening surface 19 of the mechanical closure tape tab laminate 10 with fibers upon adhering of such tape tab laminate 10 to the female fastening element 100. Such contamination may adversely affect the disposability feature of the tape tab laminate 10 and typically also results in a "fuzzying" or "stringing" of a non-release-treated exposed surface 103 of the fibrous web structure 102. In order to overcome the prior art deficiencies, the present inventions provides a method for decreasing in a closure system comprising a mechanical closure tape tab laminate 10 or a precursor of such tape tab laminate, said tape tab laminate 10 having at least one exposed adhesive fastening surface 19, and a female fastening element 100 or a precursor of such female fastening element 100, said female fastening element 100 having a fibrous web structure 102 with an exposed surface 103, the contamination of said exposed adhesive fastening surface 19 with fibers upon adhering the mechanical closure tape tab laminate to the female fastening element. The method according to the invention comprises treating of the fibrous web structure 102 with a release control component comprising one or more release control agents as is described above so that a release-treated exposed surface 103 of the fibrous web structure 102 is obtained. In a preferred embodiment of the method according to the present invention, the adhesive of the exposed adhesive fastening surface 19 and/or the release control component are preferably selected so that the formation of a two-point closure mechanism is effectively suppressed. More preferably, the adhesive of the exposed adhesive fastening surface 19 and/or the release control composition are selected so that the 90° peel adhesion between such adhesive fastening surface 19 and the exposed release-treated surface 103 of the fibrous loop structure 102 when measured according to the modified FINAT test method no. 2 described in the test section below, is less than 0.75 N/25 mm, more preferably not more than 0.5 N/25 mm and especially preferably not more than 0.25 N/25 mm.

The closure system of the present invention is preferably supplied as a kit of parts comprising the mechanical closure tape tab laminate 10 and the female fastening element 100. The tape tab laminate 10 is preferably supplied as a pre-laminated, ready-to-apply article but it is also possible that the kit of parts comprises a precursor of the tape tab laminate 10. Such precursor may comprise, for example, the single components of the tape tab laminate 10, such as the fastening tape tab 11 comprising the backing 13 bearing adhesive layer 12, the hook fastening element 15, the fingerlift 14, the secondary tape tab 30 and one or more tertiary tape tabs, with such components being laminated in-line with the manufacturing of the diaper. A method of preparing prelaminated tab laminates 10 is disclosed, for example, in EP 98 104 590.9 filed by the present Applicant on March 13, 1998. Likewise, the female fastening element 100 may be supplied as a pre-laminated article or the kit of parts may also comprise a precursor of the female fastening element 100.

The present invention is illustrated by the following examples which are intended to be explanatory but not limiting. Prior to that some test methods used in the examples will be described.

### Test methods

### 90° peel adhesion of the adhesive forming the exposed adhesive fastening surface 19

### a) from polyethylene film

The 90° peel adhesion of the adhesion forming the exposed adhesive fastening surface from a polyethylene surface is measured according to a modified standard 90° peel test method FINAT TEST METHOD NO. 2 (available from Federation Internationale des Fabricants Europeens et Transformateurs d'Adhesifs et Thermocollants sur Papiers et autres Supports). The modifications are as follows
1) FINAT 2 requires a glass substrate which has been replaced by a polyethylene film and a female fastening element, respectively, as is specified below
2) A "standard FINAT test roller" has been replaced by a 2 kg roller
3) The sample was rolled down once in each direction at a rate of 300 mm/min rather than twice at 200 mm/min as called for by the FINAT 2 method
4) The dwell time was essentially zero rather than 20 min and 24 hrs, respectively, as required by the FINAT 2 method.

The test method as used in the present invention, can be briefly described as follows:

A 25 µm thick polyethylene film having a matte surface finish (commercially available from BP, Wasserburg, Germany) was securely adhered to a 5.1 x 12.5 cm steel panel using a double coated adhesive tape (available as Tape 410 from Minnesota Mining and Manufacturing Company (3M Comp.). A 2.5 cm wide test strip of a single-sided adhesive tape was then adhered to the matte polyethylene surface and was rolled down with an automatic roller, once in each direction, using a 2,000 g roller at an approximate rate of 300 mm/min.

The panel was placed immediately (dwell time of less than one minute) into the bottom jaw of an Instron™ Model 1122 tensile tester while the end of the test strip was held by the upper jaw. The upper jaw was set in a motion at 300 mm/min while the steel panel was moved laterally so as to keep the test strip at an angle of 90° to the panel.

The force required to remove the test strip from the matte polyethylene surface was recorded and the 90° peel adhesion is reported as force/width of the test strip in N/25 mm. The test was repeated four times and the results averaged.

Further details can be taken from the FINAT test method no. 2.

### b) from the female fastening element

The procedure described in a) is repeated with the exception that the polyethylene film substrate was replaced with a strip of the female fastening element 100 held in such a configuration that the release-treated surface 103 was exposed. The force required to remove the single-sided adhesive tape test strip from release-treated surface of the fastening element was recorded and the 90° peel adhesion was reported as force/width of the test strip in N/25 mm. The test was performed four times and the results averaged.

### Example 1

A closure system comprising a mechanical closure tape tab laminate 10 according to Fig. 1 of the present patent application which is commercially available from Minnesota Mining and Manufacturing Company (3M Comp.) as Mechanical Closure Laminate M-202 comprising a polypropylene backing (thickness 115 µm) and a 25 µm thick layer of adhesive, and a female fastening element according to Fig.4 of the present application which is commercially available from 3M Comp. as EKLT-1256 Knitted Loop Tape, was used.

The tape laminate had a width of 25 mm and a length, as measured from the proximal end of the fastening tape tab to the distal end of the fingerlift, of 70 mm. The mechanical hook fastening element comprising mushroom-shaped hooks (140 hooks/cm²) had a width of 26 mm and a length of 20 mm. The exposed adhesive fastening surface which was formed by a secondary tape tab, which was distally adhered to the adhesive layer of the fastening tape tap and proximally adhered to the inside surface of the diaper though a tertiary tape tap, had a width of 25 mm and a length of 12 mm. The secondary tape tab comprising a polypropylene backing bearing a tackified synthetic rubber pressure-sensitive adhesive is commercially available from 3M Comp. as Release Tape ERT-1155.

The female fastening element had a width of 40 mm. The length of the female fastening element was chosen depending on the size of the diaper so that the exposed adhesive surface of the tab laminate did not form an adhesive closure mechanism with the outside surface of the diaper during normal use. The female fastening element comprised a warp knit, weft inserted texturized polyester fibrous web structure comprising loop elements, the loop side of which was treated with a silicone polyurea-based release coating solution to give a dry coating weight of 0.7 g/m². A polypropylene backing layer was coated onto the base layer of the fibrous web structure followed by a pressure-sensitive adhesive layer.

The 90° peel adhesion of the adhesive layer of the secondary tape tab from the release-treated loop surface of the EKLT-1256 Knitted Loop Tape and from the matte polyethylene surface, respectively, described in the test section, was measured as described above. The results are summarized below.

| **surface** | **90° peel adhesion [N/25 mm]** |
|---|---|
| matte polyethylene | 7.0 |
| EKLT-1256 Knitted Loop Tape | 1.0 |

### Example 2

Example 1 was repeated with the exception that the secondary tape tab available from 3M Comp. as Release Tape ERT-1155 was replaced with a different tape, available as ERT-1284 from 3M Comp. Release Tape ERT-1284 bears a less aggressive pressure-sensitive adhesive as compared to ERT-1155. Peel adhesion values (90°) were measured as described above from both polyethylene and release-treated loop material as described in Example 1.

| **surface** | **90° peel adhesion [N/25 mm]** |
|---|---|
| matte polyethylene | 7.0 |
| EKLT-1256 Knitted Loop Tape | 0.15 |

### Comparative Example 1

A closure system comprising a mechanical closure tape tab laminate 10 according to Fig. 1 of the present patent application which is commercially available from Minnesota Mining and Manufacturing Company (3M Co.) as Mechanical Closure Laminate M-202, and a non-release treated female fastening element according to Fig.4 of the present application which is used in the Pampers (TM) Premiums disposable diaper, commercially available from Procter & Gamble Deutschland GmbH, Schwalbach, Germany.

The 90° peel adhesion of the adhesive layer of the secondary tape tab from the non-release treated loop surface was measured as is described above. The following results were obtained:

| **surface** | **90° peel adhesion [N/25 mm]** |
|---|---|
| non-release-treated loop tape as used in Pampers (TM) Premiums diaper | 4.0 |

### Comparative Example 2

Comparative Example 1 was repeated with the exception that ERT-1155 (a component of M202 described in Example 1) was replaced with ERT-1284 bearing a tackified synthetic rubber pressure-sensitive adhesive which is less aggressive as compared to that of ERT-1155.

The 90° peel adhesion of the adhesive layer of the secondary tape tab from the non-release treated loop surface was measured as described above. The following results were obtained:

| **surface** | **90° peel adhesion [N/25 mm]** |
|---|---|
| non-release-treated loop tape as used in Pampers (TM) Premiums diaper | 1.6 |

### List of reference numbers

- 1: diaper
- 2: inside surface of the diaper
- 3: outside surface of the diaper
- 4: absorbent core
- 6: edge portion of the diaper
- 7: first end region of the diaper
- 8: second end region of the diaper
- 10: mechanical closure tape tab laminate
- 11: fastening tape tab
- 12: adhesive layer
- 13: backing
- 14: fingerlift
- 15: male mechanical fastening element
15a hook element
15b backing
- 16: cover film
- 19: exposed adhesive fastening surface
- 20: longitudinal edge of the diaper
- 30: secondary tape tab
- 31: backing
- 32: adhesive layer
- 40: tertiary tape tab
- 41: backing
- 42: adhesive layer
- 100: female fastening element
- 101: backing
- 102: fibrous web structure
- 103: outwardly facing (exposed) surtace of fibrous web structure
- 104: loop element
- 105: adhesive layer

## Claims

1. A closure system comprising a mechanical closure tape tab laminate (10) or a precursor of such tape tab laminate, and a female fastening element (100) or a precursor of such female fastening material, for an absorbent article, particularly for a disposable diaper (1), the mechanical closure tape tab laminate (10) comprising a fastening tab (11) having a backing (13) bearing an adhesive layer (12), the mechanical closure tape tab laminate (10) being attachable to the outside surface (3) of a first end region 7 of the diaper (1) or between the outside surface (3) and the inside surface (2) of the edge portion 6 of said first end region 7 of the diaper (1) through its proximal end (17) which is adjacent to its free end (18), the free end (18) of the mechanical closure tape tab laminate (10) further comprising a male mechanical fastening element (15), the mechanical closure tape tab laminate (10) having at least one exposed adhesive fastening surface (19) on the free end (18) of the fastening tape (11), the female fastening element (100) comprising a backing (101) bearing on a first major surface a fibrous web structure (102) having a release-treated exposed surface (103), the fibrous web structure (102) being capable of engaging the male fastening element (15) of the mechanical closure tape tab laminate (10), the female fastening element being attachable to the outside surface (3) of a second end region (8) of the diaper (1) opposite to said first end region (7) through a second major surface of the backing (101) to form a landing zone for the male fastening element (15) of the mechanical closure tape tab laminate (10), the release-treatment of the exposed surface (103) of the fibrous web structure (102) being selected so that the 90° peel adhesion of the exposed adhesive fastening surface (19) from the exposed surface (103) of the female fastening element (100) as measured according to the modified FINAT test method no. 2 described in the test section, is less than 1.2 N/25 mm.

2. A closure system comprising a mechanical closure tape tab laminate (10) or a precursor of such tape tab laminate, and a female fastening element (100) or a precursor of such female fastening material, for an absorbent article, particularly for a disposable diaper (1), the mechanical closure tape tab laminate (10) comprising a fastening tab (11) having a backing (13) bearing an adhesive layer (12), the mechanical closure tape tab laminate (10) being attachable to the outside surface (3) of a first end region 7 of the diaper (1) or between the outside surface (3) and the inside surface (2) of the edge portion 6 of said first end region 7 of the diaper (1) through its proximal end (17) which is adjacent to its free end (18), the free end (18) of the mechanical closure tape tab laminate (10) further comprising a male mechanical fastening element (15), the mechanical closure tape tab laminate (10) further comprising a secondary tape tab (30) having a backing (31) bearing an adhesive layer (32), the secondary tape tab (30) being attached to the free end (18) of the mechanical closure tape tab laminate (10) by means of the adhesive layer (32), by means of the adhesive layer (12) and/or by means of one or more tertiary tape tabs (40) having a backing (41) and an adhesive layer (42), the secondary tape tab (30) being attachable to the inside surface (2) of the diaper (1) by means of the adhesive layer (32) and/or by means of one or more tertiary tape tabs (40), the mechanical closure tape tab laminate (10) having at least one exposed adhesive fastening surface (19) on the free end (18) of the fastening tape (11), on the secondary tape tab (30) and/or on one or more of the tertiary tape tabs (40), the female fastening element (100) comprising a backing (101) bearing on a first major surface a fibrous web structure (102) having a release-treated exposed surface (103), the fibrous web structure (102) being capable of engaging the male fastening element (15) of the mechanical closure tape tab laminate (10), the female fastening element being attachable to the outside surface (3) of a second end region (8) of the diaper (1) opposite to said first end region (7) through a second major surface of the backing (101) to form a landing zone for the male fastening element (15) of the mechanical closure tape tab laminate (10), the release-treatment of the exposed surface (103) of the fibrous web structure (102) being selected so that the 90° peel adhesion of the exposed adhesive fastening surface (19) from the exposed surface (103) of the female fastening element (100) as measured according to the modified FINAT test method no. 2 described in the test section, is less than 1.2 N/25 mm.

3. Closure system according to any of claims 1 or 2 wherein one or more of adhesive layers (12), (32) and (42) are continuous.

4. Closure system according to any of claims 1 - 3 wherein the release -treated exposed surface (103) is obtainable by applying a release coating solution.

5. Closure system according to claim 4 wherein the release coating solution comprises one or more release agents selected from a group consisting of reactive silicones, fluorochemical polymers, epoxysilicones or radiation curable polyorganosifoxane polyurethane block copolymers.

6. Closure system according to any of claims 4 - 5 wherein the dried release coating weight is between 0.05 and 5 g/m².

7. Closure system according to any of claims 4 - 6 wherein the coating solution comprises between 1 - 25 wt.% of one or more release agents.

8. Closure system according to any of claims 1 - 7 wherein the exposed adhesive fastening surface (19) of the mechanical closure tape tab laminate (10) is essentially above the inside surface (2) of the diaper (1) and wherein the male fastening element (15) is essentially at the distal end of the tape tab laminate (10) so that the distal end of the exposed adhesive fastening surface (19) and the proximal end of the male fastening element (15) are not directly adjacent to each other.

9. Disposable absorbent article, in particular a diaper (1) comprising a closure system according to any of claims 1 - 8.

10. Method of decreasing in a closure system comprising a mechanical closure tape tab laminate (10) or a precursor of such tape tab laminate, said tape tab laminate (10) having at least one exposed adhesive fastening surface (19), and a female fastening element (100) or a precursor of such female fastening material, said female fastening element (100) comprising a fibrous web structure (102) having an exposed surface (103), the contamination of said exposed adhesive fastening surface (19) with fibers upon adhering the mechanical closure tape tab laminate (10) to the female fastening element (100), the mechanical closure tape tab laminate (10) comprising a fastening tab (11) having a backing (13) bearing an adhesive layer (12), the mechanical closure tape tab laminate (10) being attachable to the outside surface (3) of a first end region 7 of the diaper (1) or between the outside surface (3) and the inside surface (2) of the edge portion 6 of said first end region 7 of the diaper (1) through its proximal end (17) which is adjacent to its free end (18), the free end (18) of the mechanical closure tape tab laminate (10) further comprising a male mechanical fastening element (15), the mechanical closure tape tab laminate (10) having said at least one exposed adhesive fastening surface (19) on the free end (18) of the fastening tape (11), the female fastening element (100) comprising a backing (101) bearing on a first major surface said fibrous web structure (102) having said exposed surface (103), the fibrous web structure (102) being capable of engaging the male fastening element (15) of the mechanical closure tape tab laminate (10), the female fastening element (100) being attachable to the outside surface (3) of a second end region (8) of the diaper (1) opposite to said first end region (7) through a second major surface of the backing (101) to form a landing zone for the male fastening element (15) of the mechanical closure tape tab laminate (10), said method for decreasing of the contamination of the exposed adhesive fastening surface (19) with fibers upon adhering the mechanical closure tape tab laminate (10) to the female fastening element (100) comprising release-treating of the exposed surface (103) of the fibrous web structure (102), a release control component being applied to. the fibrous web structure (102) during such release-treatment being selected so that the 90° peel adhesion between the exposed adhesive fastening surface (19) and the exposed release-treated surface (103) of the female fastening element (100) as measured according to the modified FINAT test method no. 2 described in the test section, is less than 1.2 N/25 mm.

11. Method of decreasing in a closure system comprising a mechanical closure tape tab laminate (10) or a precursor of such tape tab laminate, said tape tab laminate (10) having at least one exposed adhesive fastening surface (19), and a female fastening element (100) or a precursor of such female fastening material, said female fastening element (100) comprising a fibrous web structure (102) having an exposed surface (103), the contamination of said exposed adhesive fastening surface (19) with fibers upon adhering the mechanical closure tape tab laminate (10) to the female fastening element (100), the mechanical closure tape tab laminate (10) comprising a fastening tab (11) having a backing (13) bearing an adhesive layer (12), the mechanical closure tape tab laminate (10) being attachable to the outside surface (3) of a first end region 7 of the diaper (1) or between the outside surface (3) and the inside surface (2) of the edge portion 6 of said first end region 7 of the diaper (1) through its proximal end (17) which is adjacent to its free end (18), the free end (18) of the mechanical closure tape tab laminate (10) further comprising a male mechanical fastening element (15), the mechanical closure tape tab laminate (10) further comprising a secondary tape tab (30) having a backing (31) bearing an adhesive layer (32), the secondary tape tab (30) being attached to the free end (18) of the mechanical closure tape tab laminate (10) by means of the adhesive layer (32), by means of the adhesive layer (12) and/or by means of one or more tertiary tape tabs (40) having a backing (41) and an adhesive layer (42), the secondary tape tab (30) being attachable to the inside surface (2) of the diaper (1) by means of the adhesive layer (32) and/or by means of one or more tertiary tape tabs (40), the mechanical closure tape tab laminate (10) having said at least one exposed adhesive fastening surface (19) on the free end (18) of the fastening tape (11), on the secondary tape tab (30) and/or on one or more of the tertiary tape tabs (40), the female fastening element (100) comprising a backing (101) bearing on a first major surface said fibrous web structure (102) having said exposed surface (103), the fibrous web structure (102) being capable of engaging the male fastening element (15) of the mechanical closure tape tab laminate (10), the female fastening element (100) being attachable to the outside surface (3) of a second end region (8) of the diaper (1) opposite to said first end region (7) through a second major surface of the backing (101) to form a landing zone for the male fastening element (15) of the mechanical closure tape tab laminate (10), said method for decreasing of the contamination of the exposed adhesive fastening surface (19) with fibers upon adhering the mechanical closure tape tab laminate (10) to the female fastening element (100) comprising release-treating of the exposed surface (103) of the fibrous web structure (102), a release control component being applied to the fibrous-web structure (102) during such release-treatment being selected so that the 90° peel adhesion between the exposed adhesive fastening surface (19) and the exposed release-treated surface (103) of the female fastening element (100) as measured according to the modified FINAT test method no. 2 described in the test section, is less than 1.2 N/25 mm.

12. Method according to any of claims 10 - 11 wherein one or more of adhesive layers (12), (32) and (42) are continuous.

## Patentansprüche

1. Verschluss-System, umfassend ein Laminat (10) für eine mechanische Verschlussbandlasche oder eine Vorstufe für ein solches Bandlaschenlaminat, sowie ein weibliches Befestigungselement (100) oder eine Vorstufe für ein solches weibliches Befestigungsmaterial, für einen absorbierenden Gegenstand, insbesondere für eine Wegwerfwindel (1), wobei das Laminat (10) für eine mechanische Verschlussbandlasche eine Befestigungslasche (11) mit einem Träger (13) umfasst, der eine Klebeschicht (12) trägt, das Laminat (10) für eine mechanische Verschlussbandlasche sich an der Außenfläche (3) eines ersten Endbereichs 7 der Windel (1) oder zwischen der Außenfläche (3) und der Innenfläche (2) des Randabschnitts 6 des ersten Endbereichs 7 der Windel (1) über sein proximales Ende (17) befestigen lässt, das sich neben seinem freien Ende (18) befindet, wobei das freie Ende (18) des Laminats (10) für eine mechanische Verschlussbandlasche zudem ein mechanisches männliches Befestigungselement (15) umfasst, das Laminat (10) für eine mechanische Verschlussbandlasche mindestens eine freiliegende Klebebefestigungsoberfläche (19) auf dem freien Ende (18) des Befestigungsbandes (11) aufweist, das weibliche Befestigungselement (100) einen Träger (101) umfasst, der auf einer ersten Hauptoberfläche eine faserige Gewebestruktur (102) mit einer trennmittelbehandelten freiliegenden Oberfläche (103) trägt, die faserige Gewebestruktur (102) das männliche Befestigungselement (15) des Laminats (10) für eine mechanische Verschlussbandlasche festhalten kann, das weibliche Befestigungselement sich an der Außenfläche (3) eines zweiten Endbereichs (8) der Windel (1) gegenüber dem ersten Endbereich (7) über eine zweite Hauptoberfläche des Trägers (101) befestigen lässt, so dass ein Landebereich für das männliche Befestigungselement (15) des Laminats (10) für eine mechanische Verschlussbandlasche geschaffen wird, die Trennmittelbehandlung der freiliegenden Oberfläche (103) der faserigen Gewebestruktur (102) so ausgewählt ist, dass die 90°-Schälhaftung zwischen der freiliegenden Klebebefestigungsoberfläche (19) und der freiliegenden Oberfläche (103) des weiblichen Befestigungselementes (100), wie gemäß dem im Testabschnitt beschriebenen modifizierten FINAT-Test-Verfahren Nr. 2 gemessen, kleiner als 1,2 N/25 mm ist.

2. Verschluss-System, umfassend ein Laminat (10) für eine mechanische Verschlussbandlasche oder eine Vorstufe für ein solches Bandlaschenlaminat, sowie ein weibliches Befestigungselement (100) oder eine Vorstufe für ein solches weibliches Befestigungsmaterial, für einen absorbierenden Gegenstand, insbesondere für eine Wegwerfwindel (1), wobei das Laminat (10) für eine mechanische Verschlussbandlasche eine Befestigungslasche (11) mit einem Träger (13) umfasst, der eine Klebeschicht (12) trägt, das Laminat (10) für eine mechanische Verschlussbandlasche sich an der Außenfläche (3) eines ersten Endbereichs 7 der Windel (1) oder zwischen der Außenfläche (3) und der Innenfläche (2) des Randabschnitts 6 des ersten Endbereichs 7 der Windel (1) über sein proximales Ende (17) befestigen lässt, das sich neben seinem freien Ende (18) befindet, wobei das freie Ende (18) des Laminats (10) für eine mechanische Verschlussbandlasche zudem ein mechanisches männliches Befestigungselement (15) umfasst, das Laminat (10) für eine mechanische Verschlussbandlasche zudem eine sekundäre Bandlasche (30) mit einem Träger (31) aufweist, der eine Klebeschicht (32) trägt, wobei die sekundäre Bandlasche (30) an dem freien Ende (18) des Laminats (10) für eine mechanische Verschlussbandlasche mit Hilfe der Klebeschicht (32), mit Hilfe der Klebeschicht (12) und/oder mit Hilfe von einer oder mehreren tertiären Bandlaschen (40) befestigt ist, die einen Träger (41) und eine Klebeschicht (42) aufweisen, wobei sich die sekundäre Bandlasche (30) an der Innenfläche (2) der Windel (1) mit Hilfe der Klebeschicht (32) und/oder mit Hilfe von einer oder mehreren tertiären Bandlaschen (40) befestigen lässt, wobei das Laminat (10) für eine mechanische Verschlussbandlasche mindestens eine freiliegende Klebebefestigungsoberfläche (19) auf dem freien Ende (18) des Befestigungsbandes (11), auf der sekundären Bandlasche (30) und/oder auf einer oder mehreren der tertiären Bandlaschen (40) aufweist, das weibliche Befestigungselement (100) einen Träger (101) umfasst, der auf einer ersten Hauptoberfläche eine faserige Gewebestruktur (102) mit einer trennmittelbehandelten freiliegenden Oberfläche (103) trägt, die faserige Gewebestruktur (102) das männliche Befestigungselement (15) des Laminats (10) für eine mechanische Verschlussbandlasche festhalten kann, das weibliche Befestigungselement sich an der Außenfläche (3) eines zweiten Endbereichs (8) der Windel (1) gegenüber dem ersten Endbereich (7) über eine zweite Hauptoberfläche des Trägers (101) befestigen lässt, so dass ein Landebereich für das männliches Befestigungselement (15) des Laminats (10) für eine mechanische Verschlussbandlasche geschaffen wird, die Trennmittelbehandlung der freiliegenden Oberfläche (103) der faserigen Gewebestruktur (102) so ausgewählt ist, dass die 90°-Schälhaftung zwischen der freiliegenden Klebebefestigungsoberfläche (19) und der freiliegenden Oberfläche (103) des weiblichen Befestigungselementes (100), wie gemäß dem im Testabschnitt beschriebenen modifizierten FINAT-Test-Verfahren Nr. 2 gemessen, kleiner als 1,2 N/25 mm ist.

3. Verschluss-System nach einem der Ansprüche 1 oder 2, wobei von den Klebeschichten (12), (32) und (42) eine oder mehrere kontinuierlich sind.

4. Verschluss-System nach einem der Ansprüche 1-3, wobei sich die trennmittelbehandelte freiliegende Oberfläche (103) durch Aufbringen einer Trennbeschichtungslösung erhalten lässt.

5. Verschluss-System nach Anspruch 4, wobei die Trennbeschichtungslösung ein oder mehrere Trennmittel umfasst, die aus der aus reaktiven Silikonen, fluorchemischen Polymeren, Epoxysilikonen oder strahlungshärtenden Polyorganosiloxan-Polyurethan-Blockcopolymeren bestehenden Gruppe ausgewählt werden.

6. Verschluss-System nach einem der Ansprüche 4-5, wobei das Gewicht der getrockneten Trennbeschichtung zwischen 0,05 und 5 g/m² liegt.

7. Verschluss-System nach einem der Ansprüche 4-6, wobei die Beschichtungslösung zwischen 1-25 Gew.-% von einem oder mehreren Trennmitteln umfasst.

8. Verschluss-System nach einem der Ansprüche 1-7, wobei sich die freiliegende Klebebefestigungsoberfläche (19) des Laminats (10) für eine mechanische Verschlussbandlasche im Wesentlichen über der Innenfläche (2) der Windel (1) befindet und wobei sich das männliche Befestigungselement (15) im Wesentlichen am distalen Ende des Bandlaschenlaminats (10) befindet, so dass das distale Ende der freiliegenden Klebebefestigungsoberfläche (19) und das proximale Ende des männlichen Befestigungselementes (15) einander nicht direkt benachbart sind.

9. Absorbierender Wegwerf-Gegenstand, insbesondere eine Windel (1) mit einem Verschluss-System nach einem der Ansprüche 1-8.

10. Verfahren zum Verringern in einem Verschluss-System, umfassend ein Laminat (10) für eine mechanische Verschlussbandlasche oder eine Vorstufe für ein solches Bandlaschenlaminat, wobei das Bandlaschenlaminat (10) mindestens eine freiliegende Klebebefestigungsoberfläche (19) aufweist, sowie ein weibliches Befestigungselement (100) oder eine Vorstufe für ein solches weibliches Befestigungsmaterial, wobei das weibliche Befestigungselement (100) eine faserige Gewebestruktur (102) mit einer freiliegenden Oberfläche (103) umfasst, der Verunreinigung der freiliegenden Klebebefestigungsoberfläche (19) mit Fasern beim Befestigen des Laminats (10) für eine mechanische Verschlussbandlasche an einem weiblichen Befestigungselement (100), wobei das Laminat (10) für eine mechanische Verschlussbandlasche eine Befestigungslasche (11) mit einem Träger (13) umfasst, der eine Klebeschicht (12) trägt, das Laminat (10) für eine mechanische Verschlussbandlasche sich an der Außenfläche (3) eines ersten Endbereichs 7 der Windel (1) oder zwischen der Außenfläche (3) und der Innenfläche (2) des Randabschnitts 6 des ersten Endbereichs 7 der Windel (1) über sein proximales Ende (17) befestigen lässt, das sich neben seinem freien Ende (18) befindet, wobei das freie Ende (18) des Laminats (10) für eine mechanische Verschlussbandlasche zudem ein mechanisches männliches Befestigungselement (15) umfasst, das Laminat (10) für eine mechanische Verschlussbandlasche mindestens eine freiliegende Klebebefestigungsoberfläche (19) auf dem freien Ende (18) des Befestigungsbandes (11) aufweist, das weibliche Befestigungselement (100) einen Träger (101) umfasst, der auf einer ersten Hauptoberfläche eine faserige Gewebestruktur (102) mit der freiliegenden Oberfläche (103) trägt, die faserige Gewebestruktur (102) das männliche Befestigungselement (15) des Laminats (10) für eine mechanische Verschlussbandlasche festhalten kann, das weibliche Befestigungselement (100) sich an der Außenfläche (3) eines zweiten Endbereichs (8) der Windel (1) gegenüber dem ersten Endbereich (7) über eine zweite Hauptoberfläche des Trägers (101) befestigen lässt, so dass ein Landebereich für das männliche Befestigungselement (15) des Laminats (10) für eine mechanische Verschlussbandlasche geschaffen wird, das Verfahren zum Verringern der Verunreinigung der freiliegenden Klebebefestigungsoberfläche (19) mit Fasern beim Befestigen des Laminats (10) für eine mechanische Verschlussbandlasche an dem weiblichen Befestigungselement (100) die Trennmittelbehandlung der freiliegenden Oberfläche (103) der faserigen Gewebestruktur (102) umfasst, eine Trennmittel-Kontrollkomponente auf die faserige Gewebestruktur (102) während einer solchen Trennmittelbehandlung aufgebracht wird, die so ausgewählt ist, dass die 90°-Schälhaftung zwischen der freiliegenden Klebebefestigungsoberfläche (19) und der freiliegenden trennmittelbehandelten Oberfläche (103) des weiblichen Befestigungselementes (100), wie gemäß dem im Testabschnitt beschriebenen modifizierten FINAT-Test-Verfahren Nr. 2 gemessen, kleiner als 1,2 N/25 mm ist.

11. Verfahren zum Verringern in einem Verschluss-System, umfassend ein Laminat (10) für eine mechanische Verschlussbandlasche oder eine Vorstufe für ein solches Bandlaschenlaminat, wobei das Bandlaschenlaminat (10) mindestens eine freiliegende Klebebefestigungsoberfläche (19) aufweist, sowie ein weibliches Befestigungselement (100) oder eine Vorstufe für ein solches weibliches Befestigungsmaterial, wobei das weibliche Befestigungselement (100) eine faserige Gewebestruktur (102) mit einer freiliegenden Oberfläche (103) umfasst, der Verunreinigung der freiliegenden Klebebefestigungsoberfläche (19) mit Fasern beim Befestigen des Laminats (10) für eine mechanische Verschlussbandlasche an dem weiblichen Befestigungselement (100), wobei das Laminat (10) für eine mechanische Verschlussbandlasche eine Befestigungslasche (11) mit einem Träger (13) umfasst, der eine Klebeschicht (12) trägt, das Laminat (10) für eine mechanische Verschlussbandlasche sich an der Außenfläche (3) eines ersten Endbereichs 7 der Windel (1) oder zwischen der Außenfläche (3) und der Innenfläche (2) des Randabschnitts 6 des ersten Endbereichs 7 der Windel (1) über sein proximales Ende (17) befestigen lässt, das sich neben seinem freien Ende (18) befindet, wobei das freie Ende (18) des Laminats (10) für eine mechanische Verschlussbandlasche zudem ein mechanisches männliches Befestigungselement (15) umfasst, das Laminat (10) für eine mechanische Verschlussbandlasche zudem eine sekundäre Bandlasche (30) mit einem Träger (31) aufweist, der eine Klebeschicht (32) trägt, wobei die sekundäre Bandlasche (30) an dem freien Ende (18) des Laminats (10) für eine mechanische Verschlussbandlasche mit Hilfe der Klebeschicht (32), mit Hilfe der Klebeschicht (12) und/oder mit Hilfe von einer oder mehreren tertiären Bandlaschen (40) befestigt ist, die einen Träger (41) und eine Klebeschicht (42) aufweisen, wobei sich die sekundäre Bandlasche (30) an der Innenfläche (2) der Windel (1) mit Hilfe der Klebeschicht (32) und/oder mit Hilfe von einer oder mehreren tertiären Bandlaschen (40) befestigen lässt, wobei das Laminat (10) für eine mechanische Verschlussbandlasche mindestens eine freiliegende Klebebefestigungsoberfläche (19) auf dem freien Ende (18) des Befestigungsbandes (11), auf der sekundären Bandlasche (30) und/oder auf einer oder mehreren der tertiären Bandlaschen (40) aufweist, das weibliche Befestigungselement (100) einen Träger (101) umfasst, der auf einer ersten Hauptoberfläche die faserige Gewebestruktur (102) mit einer freiliegenden Oberfläche (103) trägt, die faserige Gewebestruktur (102) das männliche Befestigungselement (15) des Laminats (10) für eine mechanische Verschlussbandlasche festhalten kann, das weibliche Befestigungselement (100) sich an der Außenfläche (3) eines zweiten Endbereichs (8) der Windel (1) gegenüber dem ersten Endbereich (7) über eine zweite Hauptoberfläche des Trägers (101) befestigen lässt, so dass ein Landebereich für das männliche Befestigungselement (15) des Laminats (10) für eine mechanische Verschlussbandlasche geschaffen wird, das Verfahren zum Verringern der Verunreinigung der freiliegenden Klebebefestigungsoberfläche (19) mit Fasern beim Befestigen des Laminats (10) für eine mechanische Verschlussbandlasche an dem weiblichen Befestigungselement (100) die Trennmittelbehandlung der freiliegenden Oberfläche (103) der faserigen Gewebestruktur (102) umfasst, eine Trennmittel-Kontrollkomponente auf die faserige Gewebestruktur (102) während einer solchen Trennmittelbehandlung aufgebracht wird, die so ausgewählt ist, dass die 90°-Schälhaftung zwischen der freiliegenden Klebebefestigungsoberfläche (19) und der freiliegenden trennmittelbehandelten Oberfläche (103) des weiblichen Befestigungselementes (100), wie gemäß dem im Testabschnitt beschriebenen modifizierten FINAT-Test-Verfahren Nr. 2 gemessen, kleiner als 1,2 N/25 mm ist.

12. Verfahren nach einem der Ansprüche 10-11, wobei von den Klebeschichten (12), (32) und (42) eine oder mehrere kontinuierlich sind.

## Revendications

1. Système de fermeture comprenant un stratifié en forme de languette permettant une fermeture mécanique (10) ou un précurseur d'un tel stratifié en forme de languette, et un élément de fixation femelle (100) ou un précurseur d'un tel matériau de fixation femelle, pour un article absorbant, en particulier pour une couche à jeter après usage (1), le stratifié en forme de languette permettant une fermeture mécanique (10) comprenant une patte de fixation (11) comportant un support (13) portant une couche d'adhésif (12), le stratifié en forme de languette permettant une fermeture mécanique (10) étant susceptible d'être attaché à la surface extérieure (3) d'une première région d'extrémité 7 de la couche (1) ou entre la surface extérieure (3) et la surface intérieure (2) de la bordure 6 de ladite première région d'extrémité 7 de la couche (1) par son extrémité proximale (17) qui est adjacente à son extrémité libre (18), l'extrémité libre (18) du stratifié en forme de languette permettant une fermeture mécanique (10) comprenant en outre un élément de fixation mécanique mâle (15), le stratifié en forme de languette permettant une fermeture mécanique (10) comportant au moins une surface de fixation adhésive exposée (19) sur l'extrémité libre (18) du ruban de fixation (11), l'élément de fixation femelle (100) comprenant un support (101) portant, sur une première surface principale, une structure de bande fibreuse (102) comportant une surface exposée ayant été soumise à un traitement antiadhésif (103), la structure de bande fibreuse (102) étant capable de venir en prise avec l'élément de fixation mâle (15) du stratifié en forme de languette permettant une fermeture mécanique (10), l'élément de fixation femelle étant susceptible d'être attaché à la surface extérieure (3) d'une deuxième région d'extrémité (8) de la couche (1) opposée à ladite première région d'extrémité (7) par l'intermédiaire d'une seconde surface principale du support (101) pour former une zone d'application pour l'élément de fixation mâle (15) du stratifié en forme de languette permettant une fermeture mécanique (10), le traitement antiadhésif de la surface exposée (103) de la structure de bande fibreuse (102) étant choisi de telle sorte que l'adhérence au pelage à 90° de la surface de fixation adhésive exposée (19) sur la surface exposée (103) de l'élément de fixation femelle (100), mesurée selon la méthode d'essai FINAT modifiée n° 2 décrite dans le paragraphe sur les essais, soit inférieure à 1,2 N/25 mm.

2. Système de fermeture comprenant un stratifié en forme de languette permettant une fermeture mécanique (10) ou un précurseur d'un tel stratifié en forme de languette, et un élément de fixation femelle (100) ou un précurseur d'un tel matériau de fixation femelle, pour un article absorbant, en particulier pour une couche à jeter après usage (1), le stratifié en forme de languette permettant une fermeture mécanique (10) comprenant une patte de fixation (11) comportant un support (13) portant une couche d'adhésif (12), le stratifié en forme de languette permettant une fermeture mécanique (10) étant susceptible d'être attaché à la surface extérieure (3) d'une première région d'extrémité 7 de la couche (1) ou entre la surface extérieure (3) et la surface intérieure (2) de la bordure 6 de ladite première région d'extrémité 7 de la couche (1) par son extrémité proximale (17) qui est adjacente à son extrémité libre (18), l'extrémité libre (18) du stratifié en forme de languette permettant une fermeture mécanique (10) comprenant en outre un élément de fixation mécanique mâle (15), le stratifié en forme de languette permettant une fermeture mécanique (10) comprenant en outre une languette secondaire (30) munie d'un support (31) portant une couche d'adhésif (32), la languette secondaire (30) étant attachée à l'extrémité libre (18) du stratifié en forme de languette permettant une fermeture mécanique (10) au moyen de la couche d'adhésif (32), au moyen de la couche d'adhésif (12) et/ou au moyen d'une ou plusieurs languettes tertiaires (40) comportant un support (41) et une couche d'adhésif (42), la languette secondaire (30) pouvant être attachée à la surface intérieure (2) de la couche (1) au moyen de la couche d'adhésif (32) et/ou au moyen d'une ou plusieurs languettes tertiaires (40), le stratifié en forme de languette permettant une fermeture mécanique (10) présentant au moins une surface de fixation adhésive exposée (19) sur l'extrémité libre (18) du ruban de fixation (11), sur la languette secondaire (30) et/ou sur une ou plusieurs des languettes tertiaires (40), l'élément de fixation femelle (100) comprenant un support (101) portant, sur une première surface principale, une structure de bande fibreuse (102) présentant une surface exposée ayant été soumise à un traitement antiadhésif (103), la structure de bande fibreuse (102) étant capable de venir en prise avec l'élément de fixation mâle (15) du stratifié en forme de languette permettant une fermeture mécanique (10), l'élément de fixation femelle étant susceptible d'être attaché à la surface extérieure (3) d'une deuxième région d'extrémité (8) de la couche (1) opposée à ladite première région d'extrémité (7) par l'intermédiaire d'une seconde surface principale du support (101) pour former une zone d'application pour l'élément de fixation mâle (15) du stratifié en forme de languette permettant une fermeture mécanique (10), le traitement antiadhésif de la surface exposée (103) de la structure de bande fibreuse (102) étant choisi de telle sorte que l'adhérence au pelage à 90° de la surface de fixation adhésive exposée (19) sur la surface exposée (103) de l'élément de fixation femelle (100), mesurée selon la méthode d'essai FINAT modifiée n° 2 décrite dans le paragraphe sur les essais, soit inférieure à 1,2 N/25 mm.

3. Système de fermeture selon l'une quelconque des revendications 1 ou 2 dans lequel une ou plusieurs des couches adhésives (12), (32) et (42) sont continues.

4. Système de fermeture selon l'une quelconque des revendications 1 - 3 dans lequel la surface exposée ayant été soumise à un traitement antiadhésif (103) peut être obtenue par application d'une solution de revêtement antiadhésif.

5. Système de fermeture selon la revendication 4 dans lequel la solution de revêtement antiadhésif comprend un ou plusieurs agents antiadhésifs choisis dans un groupe constitué par les silicones réactives, les polymères fluorés, les époxysilicones ou les copolymères séquencés polyorganosiloxane polyuréthane durcissables par un rayonnement.

6. Système de fermeture selon l'une quelconque des revendications 4 - 5 dans lequel le poids sec du revêtement antiadhésif est compris entre 0,05 et 5 g/m².

7. Système de fermeture selon l'une quelconque des revendications 4 - 6 dans lequel la solution de revêtement comprend entre 1 - 25% en poids d'un ou de plusieurs agents antiadhésifs.

8. Système de fermeture selon l'une quelconque des revendications 1 - 7 dans lequel la surface de fixation adhésive exposée (19) du stratifié en forme de languette permettant une fermeture mécanique (10) est essentiellement située au-dessus de la surface intérieure (2) de la couche (1) et dans lequel l'élément de fixation mâle (15) est essentiellement situé au niveau de l'extrémité distale du stratifié en forme de languette (10) de telle sorte que l'extrémité distale de la surface de fixation adhésive exposée (19) et l'extrémité proximale de l'élément de fixation mâle (15) ne soient pas directement adjacentes l'une à l'autre.

9. Article absorbant à jeter après usage, en particulier une couche (1), comprenant un système de fermeture selon l'une quelconque des revendications 1 - 8.

10. Procédé permettant de diminuer, dans un système de fermeture comprenant un stratifié en forme de languette permettant une fermeture mécanique (10) ou un précurseur d'un tel stratifié en forme de languette, ledit stratifié en forme de languette (10) comportant au moins une surface de fixation adhésive exposée (19), et un élément de fixation femelle (100) ou un précurseur d'un tel matériau de fixation femelle, ledit élément de fixation femelle (100) comprenant une structure de bande fibreuse (102) présentant une surface exposée (103), la contamination de ladite surface de fixation adhésive exposée (19) par des fibres, au moment où l'on colle le stratifié en forme de languette permettant une fermeture mécanique (10) à l'élément de fixation femelle (100), le stratifié en forme de languette permettant une fermeture mécanique (10) comprenant une patte de fixation (11) comportant un support (13) portant une couche d'adhésif (12), le stratifié en forme de languette permettant une fermeture mécanique (10) étant susceptible d'être attaché à la surface extérieure (3) d'une première région d'extrémité 7 de la couche (1) ou entre la surface extérieure (3) et la surface intérieure (2) de la bordure 6 de ladite première région d'extrémité 7 de la couche (1) par son extrémité proximale (17) qui est adjacente à son extrémité libre (18), l'extrémité libre (18) du stratifié en forme de languette permettant une fermeture mécanique (10) comprenant en outre un élément de fixation mécanique mâle (15), le stratifié en forme de languette permettant une fermeture mécanique (10) présentant ladite au moins une surface de fixation adhésive exposée (19) sur l'extrémité libre (18) du ruban de fixation (11), l'élément de fixation femelle (100) comprenant un support (101) portant, sur une première surface principale, ladite structure de bande fibreuse (102) présentant ladite surface exposée (103), la structure de bande fibreuse (102) étant capable de venir en prise avec l'élément de fixation mâle (15) du stratifié en forme de languette permettant une fermeture mécanique (10), l'élément de fixation femelle (100) étant susceptible d'être attaché à la surface extérieure (3) d'une deuxième région d'extrémité (8) de la couche (1) opposée à ladite première région d'extrémité (7) par l'intermédiaire d'une seconde surface principale du support (101) pour former une zone d'application pour l'élément de fixation mâle (15) du stratifié en forme de languette permettant une fermeture mécanique (10), ledit procédé permettant de diminuer la contamination de la surface de fixation adhésive exposée (19) par des fibres au moment où l'on colle le stratifié en forme de languette permettant une fermeture mécanique (10) à l'élément de fixation femelle (100) comprenant le traitement antiadhésif de la surface exposée (103) de la structure de bande fibreuse (102), un élément de réglage de l'anti-adhérence étant appliqué sur la structure de bande fibreuse (102) durant un tel traitement antiadhésif et étant choisi de telle sorte que l'adhérence au pelage à 90° entre la surface de fixation adhésive exposée (19) et la surface exposée ayant été soumise à un traitement antiadhésif (103) de l'élément de fixation femelle (100), mesurée selon la méthode d'essai FINAT modifiée n° 2 décrite dans le paragraphe sur les essais, soit inférieure à 1,2 N/25 mm.

11. Procédé permettant de diminuer, dans un système de fermeture comprenant un stratifié en forme de languette permettant une fermeture mécanique (10) ou un précurseur d'un tel stratifié en forme de languette, ledit stratifié en forme de languette (10) comportant au moins une surface de fixation adhésive exposée (19), et un élément de fixation femelle (100) ou un précurseur d'un tel matériau de fixation femelle, ledit élément de fixation femelle (100) comprenant une structure de bande fibreuse (102) présentant une surface exposée (103), la contamination de ladite surface de fixation adhésive exposée (19) par des fibres, au moment où l'on colle le stratifié en forme de languette permettant une fermeture mécanique (10) à l'élément de fixation femelle (100), le stratifié en forme de languette permettant une fermeture mécanique (10) comprenant une patte de fixation (11) comportant un support (13) portant une couche d'adhésif (12), le stratifié en forme de languette permettant une fermeture mécanique (10) étant susceptible d'être attaché à la surface extérieure (3) d'une première région d'extrémité 7 de la couche (1) ou entre la surface extérieure (3) et la surface intérieure (2) de la bordure 6 de ladite première région d'extrémité 7 de la couche (1) par son extrémité proximale (17) qui est adjacente à son extrémité libre (18), l'extrémité libre (18) du stratifié en forme de languette permettant une fermeture mécanique (10) comprenant en outre un élément de fixation mécanique mâle (15), le stratifié en forme de languette permettant une fermeture mécanique (10) comprenant en outre une languette secondaire (30) comportant un support (31) portant une couche d'adhésif (32), la languette secondaire (30) étant attachée à l'extrémité libre (18) du stratifié en forme de languette permettant une fermeture mécanique (10) au moyen de la couche d'adhésif (32), au moyen de la couche d'adhésif (12) et/ou au moyen d'une ou plusieurs languettes tertiaires (40) comportant un support (41) et une couche d'adhésif (42), la languette secondaire (30) étant susceptible d'être attachée à la surface intérieure (2) de la couche (1) au moyen de la couche d'adhésif (32) et/ou au moyen d'une ou de plusieurs languettes tertiaires (40), le stratifié en forme de languette permettant une fermeture mécanique (10) présentant ladite au moins une surface de fixation adhésive exposée (19) sur l'extrémité libre (18) du ruban de fixation (11), sur la languette secondaire (30) et/ou sur une ou plusieurs des languettes tertiaires (40), l'élément de fixation femelle (100) comprenant un support (101) portant, sur une première surface principale, ladite structure de bande fibreuse (102) présentant ladite surface exposée (103), la structure de bande fibreuse (102) étant capable de venir en prise avec l'élément de fixation mâle (15) du stratifié en forme de languette permettant une fermeture mécanique (10), l'élément de fixation femelle (100) étant susceptible d'être attaché à la surface extérieure (3) d'une deuxième région d'extrémité (8) de la couche (1) opposée à ladite première région d'extrémité (7) par l'intermédiaire d'une seconde surface principale du support (101) pour former une zone d'application pour l'élément de fixation mâle (15) du stratifié en forme de languette permettant une fermeture mécanique (10), ledit procédé permettant de diminuer la contamination de la surface de fixation adhésive exposée (19) par des fibres au moment où l'on colle le stratifié en forme de languette permettant une fermeture mécanique (10) à l'élément de fixation femelle (100) comprenant le traitement antiadhésif de la surface exposée (103) de la structure de bande fibreuse (102), un élément de réglage de l'anti-adhérence étant appliqué sur la structure de bande fibreuse (102) durant un tel traitement antiadhésif et étant choisi de telle sorte que l'adhérence au pelage à 90° entre la surface de fixation adhésive exposée (19) et la surface exposée ayant été soumise à un traitement antiadhésif 103) de l'élément de fixation femelle (100), mesurée selon la méthode d'essai FINAT modifiée n° 2 décrite dans le paragraphe sur les essais, soit inférieure à 1,2 N/25 mm.

12. Procédé selon l'une quelconque des revendications 10 - 11 dans lequel une ou plusieurs des couches adhésives (12), (32) et (42) sont continues.
